# EUROPEAN PATENT APPLICATION

(11) **EP 2 377 518 A1**
(43) Date of publication of application: **19.10.2011**
(21) Application number: 11159400.8
(22) Date of filing: 23.03.2011
(51) Int. Cl.: A61K 9/16, A61K 9/20, A61K 9/48

(54) **Stable aliskiren formulations**

(30) Priority: 24.03.2010 TR 201002256
(71) Applicant: Sanovel Ilac Sanayi ve Ticaret A.S., 34398 Istanbul (TR)
(72) Inventor: Cifter, Ümit, 34398, Istanbul (TR); Türkyilmaz, Ali, 34398, Istanbul (TR); Yelken, Gülay, 34398, Istanbul (TR)
(74) Representative: Sevinç, Erkan

(57) **Abstract**

An oral pharmaceutical formulation, comprising croscarmellose sodium and internal granules comprising a pharmaceutically acceptable salt or polymorph of aliskiren and stearic acid.

## Description

### Field of Invention

The present invention relates to formulations of aliskiren or a pharmaceutically acceptable salt of aliskiren. The present invention more particularly relates to stable formulations of aliskiren with desired levels of solubility and dissolution rate.

### Background of Invention

Aliskiren, with the chemical name (2S,4S,5S,7S)-5-amino-N-(2-carbamoyl-2-methylpropyl)-4-hydroxy-2-isopropyl-7-[4-methoxy-3-(3-methoxypropoxy)benzyl]-8-methylnonamide, is the first orally-active renin inhibitor with a non-peptide structure. Its chemical structure is illustrated with Formula I given below.

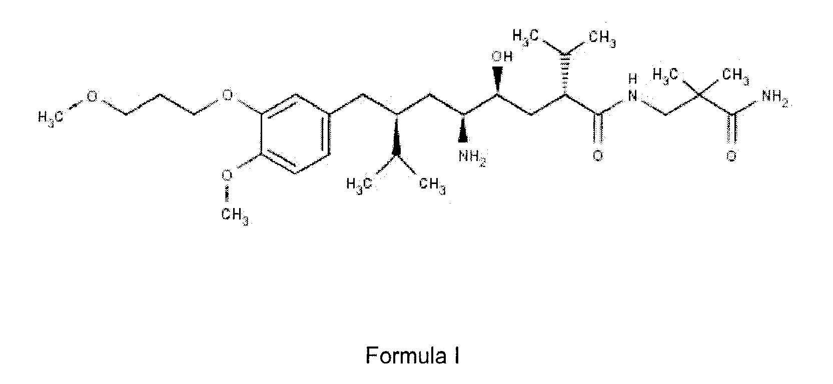

The patent US5559111 discloses the aliskiren molecule together with δ-amino-y-hydroxy-ω-aryl-alkanoic acid amide derivatives.

The patent EP1200390 discloses intermediates in preparing the aliskiren molecule and processes for their preparation.

The patent application WO2009143423A1 discloses aliskiren monofumarate and a process for the preparation thereof.

The patent application W02009149344 discloses a solid state of aliskiren free base.

The patent application W02009040373 discloses a roller compacted solid oral dosage form comprising aliskiren in an amount of more than 38% by weight based on the total weight of the dosage form.

The patent application W02005058291 discloses pharmaceutical compositions for oral administration comprising a renin inhibitor in an absorption enhancing carrier medium containing a lipophilic component, a high HLB surfactant, and a hydrophilic component, said composition upon admixing forming a stable microemulsion preconcentrate.

Aliskiren hemifumarate is a white to bright yellowish crystalline powder. It is freely soluble in phosphate buffer, n-octanol, and water. Due to its physicochemical structure, however, it is quite difficult to obtain a stable oral formulation of aliskiren. Water contact of aliskiren results in changes in the polymorphic structure, frequently leading to stability problems.

Use of water during aliskiren granulation seems to be unfeasible. Additionally, organic solvents used for granulation purposes give rise to solubility issues, as well as to issues of dissolution in final formulations. This fact is unwanted with respect to both producers and users.

Considering such problems, it is obvious that a novelty is required in the relevant art of formulations comprising aliskiren.

### Object and Brief Description of Invention

The present invention provides an aliskiren formulation, eliminating all aforesaid problems and bringing additional advantages to the relevant prior art.

Accordingly, the main object of the present invention is to obtain a formulation of aliskiren, which is stable and has a desired level of solubility and dissolution rate.

Another object of the present invention is to develop a process, enabling to produce aliskiren without influencing the solubility and dissolution rate thereof.

A further object of the present invention is to obtain various combination formulations of aliskiren.

A pharmaceutical formulation comprising aliskiren or a pharmaceutically acceptable salt or polymorph of aliskiren has been developed to carry out all objects, referred to above and to emerge from the following detailed description.

According to a preferred embodiment of the present invention, said novelty is carried out with croscarmellose sodium and internal granules, containing a pharmaceutically acceptable salt or polymorph of aliskiren and stearic acid.

According to a preferred embodiment of the present invention, the weight proportion of stearic acid to croscarmellose sodium is between 0.05 to 25.

According to another preferred embodiment of the present invention, the weight proportion of stearic acid to croscarmellose sodium is between 0.05 to 20.

According to a further preferred embodiment of the present invention, the weight proportion of stearic acid to croscarmellose sodium is between 0.09 to 15.

In a preferred embodiment according to the present invention, the subject formulation further comprises valsartan and hydrochlorothiazide as active agent.

In a preferred embodiment according to the present invention, at least one or a mixture of the following is/are used as a diluent: lactose, microcrystalline cellulose, cornstarch, mannitol, calcium phosphate anhydrate, dibasic calcium phosphate dihydrate, calcium phosphate trihydrate, sugars, sorbitol, mannitol, xylitol, sucrose polysaccharides. The diluent used is preferably cornstarch.

In a preferred embodiment according to the present invention, further comprises disintegrant or super-disintegrant: sodium starch glycolate, crospovidone, sodium alginate, glue, starch, pregelatinized starch, magnesium aluminum silicate, microcrystalline cellulose. Croscarmellose sodium or other disintegrants may either be provided in internal or external granules, or some part thereof in internal granules, and the remaining in external granules.

A preferred embodiment according to the present invention further comprises colloidal silicone dioxide, talk, or aluminum silicate as a glidant. The glidant used is preferably colloidal silicone dioxide.

A preferred embodiment according to the present invention comprises magnesium stearate as a lubricant.

A further preferred embodiment according to the present invention provides a method for preparing a pharmaceutical formulation, this method comprising the steps of
a. melting stearic acid at 80°C,
b. adding melted stearic acid into a powder mixture of aliskiren and mixing this mixture to provide granules,
c. drying the granules,
d. sieving dried granules,
e. adding the croscarmellose sodium, cornstarch and colloidal silicone dioxide and mixing the resulting mixture until a uniform mixture is obtained,
f. sieving magnesium stearate, adding it into this mixture, and mixing the resultant mixture shortly,
g. compressing the final form of powder mixture from the preceding step into tablets, or filling the same powder mixture into capsules.

In a further preferred embodiment of the present invention, said pharmaceutical formulation contains the following ingredients only:
a. aliskiren or a pharmaceutically acceptable salt or polymorph thereof at 5 to 85% by weight,
b. stearic acid at 0.1 to 25% by weight,
c. croscarmellose sodium at 0.1 to 25% by weight,
d. cornstarch at 5 to 90% by weight,
e. colloidal silicone dioxide at 0.1 to 10% by weight, and
f. magnesium stearate at 0.1 to 5% by weight.

### Detailed Description of Invention

### Example 1

| ***Unit Formula*** | ***amount %*** |
|---|---|
| **Intragranular Ingredients** | |
| Aliskiren hemifumarate | 5-85 |
| Stearic acid | 0.1-25 |

| **Extragranular Ingredients** | |
|---|---|
| Croscarmellose sodium | 0.1-25 |
| Cornstarch | 5-90 |
| Colloidal silicium dioxide | 0.1-10 |
| Magnesium stearate | 0.1-10 |

### Example 2

| ***Unit Formula*** | ***amount %*** |
|---|---|
| **Intragranular Ingredients** | |
| Aliskiren hemifumarate | 5-85 |
| Valsartan | 5- 70 |
| Stearic acid | 0.1-25 |

| **Extragranular Ingredients** | |
|---|---|
| Croscarmellose sodium | 0.1-25 |
| Cornstarch | 5-90 |
| Colloidal silicium dioxide | 0.1-10 |
| Magnesium stearate | 0.1-10 |

### Example 3

| ***Unit Formula*** | ***amount %*** |
|---|---|
| **Intragranular Ingredients** | |
| Aliskiren hemifumarate | 5-85 |
| Hydrochlorothiazide | 1- 7.5 |
| Stearic acid | 0.1-25 |

| **Extragranular Ingredients** | |
|---|---|
| Croscarmellose sodium | 0.1-25 |
| Cornstarch | 5-90 |
| Colloidal silicium dioxide | 0.1-10 |
| Magnesium stearate | 0.1-10 |

Stearic acid is melted at 80°C. Melted stearic acid is added into a powder of aliskiren and this mixture is mixed to provide granules. Then the granules are sieved. The croscarmellose sodium, cornstarch and colloidal silicone dioxide are then added, thereafter the resulting mixture is mixed until a uniform mixture is obtained. Magnesium stearate is sieved and added into this mixture, and the resultant mixture is mixed for a short period of time. The final form of powder mixture is compressed into tablets, or filled into capsules. Another active ingredients such as valsartan or hydrochlorothiazide is added extragranular phase.

Stearic acid is a hydrophobic binding agent with very low viscosity, which can be melted. Using stearic acid allows obtaining granules with more porous structures. Since the granules have low viscosity, their expansion mechanism occurs with the simultaneous occurrence of breaking and joining processes of agglomerates. Thus, mechanically stable and uniform granules are obtained with a lower particle size distribution.

With the invention realized, stable aliskiren formulations can be obtained which have surprisingly good solubility and dissolution rates, and thus high bioavailability. With the formulation developed, desired results are obtained with croscarmellose sodium and internal granules comprising a pharmaceutically acceptable salt or polymorph of aliskiren and stearic acid.

It is also possible to use the followings in place of stearic acid: Poloxamer 188 (Polyoxyethylene-Polyoxypropylene block copolymer), gelucire 50/13 (Stearyl Macrogolglyceride), polyethylene glycol, Stearate 6000, all waxes (microcrystalline wax, paraffin wax), glyceryl behenate, glyceryl monostearate, glyceryl palmitostearate, glyceryl stearate, hydrogenated castor oil, stearic alcohol.

This formulation provides the treatment of hypertension.

It is further possible to use the following additional auxiliaries in the formulation.

Suitable binders include, but are not restricted to, at least one or a mixture of polyvinylprolidone, gelatin, sugars, glucose, natural gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to, at least one or a mixture of colloidal silicone dioxide, talk, aluminum silicate.

Suitable lubricants include, but are not restricted to, at least one or a mixture of sodium stearil fumarat, magnesium stearat, polyethylene glycol, stearic acid, metal stearates, boric acid, sodium chloride benzoate and acetate, sodium or magnesium lauryl sulfate.

Suitable preservatives include, but are not restricted to, at least one or a mixture of methyl paraben and propyl paraben and salts thereof (e.g. sodium or potassium salts), sodium benzoate, citric acid, benzoik acid, butylated hydroxytoluene and butylated hydroxyanisole.

Suitable surface active agents include, but are not restricted to, at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate.

Suitable coating agents include, but are not restricted to hydroxypropyl methyl cellulose, polyethylene glycol, polyvinylprolidone, polyvinylprolidone-vinyl acetate copolymer(PVP-VA), polyvinyl alcohol like polymers, and all kinds of OpadryTM, as well as pigments, dyes, titanium dioxide and iron oxide, talk.

The protection scope of the present invention is set forth in the annexed Claims and cannot be restricted to the illustrative disclosures given above, under the detailed description. Any alternative embodiments to be produced by persons skilled in the art according to the basic principles, which are under the protection scope as set forth in the Claims, shall be an infringement of the present invention.

## Claims

1. An oral pharmaceutical formulation, comprising croscarmellose sodium and internal granules comprising a pharmaceutically acceptable salt or polymorph of aliskiren and stearic acid in which the weight proportion of stearic acid to croscarmellose sodium is in the range of 0.05 to 25.

2. The pharmaceutical formulation according to Claim 1, wherein the weight proportion of stearic acid to croscarmellose sodium is in the range of 0.05 to 20.

3. The pharmaceutical formulation according to any of the preceding claims, wherein the weight proportion of stearic acid to croscarmellose sodium is in the range of 0.09 to 15.

4. The pharmaceutical formulation according to any of the preceding claims, further comprising valsartan and hydrochlorothiazide as an active agent.

5. The pharmaceutical formulation according to any of the preceding claims, wherein the further excipient as a diluent used comprises at least one or a mixture of lactose, microcrystalline cellulose, cornstarch, mannitol, calcium phosphate anhydrate, dibasic calcium phosphate dihydrate, calcium phosphate trihydrate, sugars, sorbitol, mannitol, xylitol, sucrose polysaccharides.

6. The pharmaceutical formulation according to any of the preceding claims, wherein said diluent is cornstarch.

7. The pharmaceutical formulation according to any of the preceding claims, further comprising sodium starch glycolate, crospovidone, sodium alginate, glue, starch, magnesium aluminum silicate as a disintegrant or super-disintegrant.

8. The pharmaceutical formulation according to any of the preceding claims, further comprising colloidal silicone dioxide, talk or aluminum silicate as a glidant.

9. The pharmaceutical formulation according to any of the preceding claims, further wherein said glidant is colloidal silicone dioxide.

10. The pharmaceutical formulation according to any of the preceding claims, comprising magnesium stearate as a lubricant.

11. A method for preparing a pharmaceutical formulation according to any of the preceding claims, comprising the steps of
a. melting stearic acid at 80°C,
b. adding melted stearic acid into a powder mixture of aliskiren and mixing this mixture to provide granules,
c. drying the granules,
d. sieving dried granules,
e. adding the croscarmellose sodium, cornstarch and colloidal silicone dioxide and mixing the resulting mixture until a uniform mixture is obtained,
f. sieving magnesium stearate, adding it into this mixture, and mixing the resultant mixture shortly,
g. compressing the final form of powder mixture from the preceding step into tablets, or filling the same powder mixture into capsules.

12. The pharmaceutical formulation according to any of the preceding claims, consist of
a. aliskiren or a pharmaceutically acceptable salt or polymorph thereof at 5 to 85% by weight,
b. stearic acid at 0.1 to 25% by weight,
c. croscarmellose sodium at 0.1 to 25% by weight,
d. cornstarch at 5 to 90% by weight,
e. colloidal silicone dioxide at 0.1 to 10% by weight, and
f. magnesium stearate at 0.1 to 5% by weight.

13. The pharmaceutical formulation according to any of the preceding claims for use in the prevention or treatment of hypertension in mammalians, particularly in humans.
